# EUROPÄISCHE PATENTSCHRIFT

(11) **EP 2 397 178 B1**
(45) Veröffentlichungstag und Bekanntmachung des Hinweises auf die Patenterteilung: **27.11.2013**
(21) Anmeldenummer: 11168488.2
(22) Anmeldetag: 01.06.2011
(51) Int. Cl.: A61M 15/00

(54) **Inhalationsvorrichtung**
Inhalation device
Dispositif d'inhalation

(30) Priorität: 15.06.2010 DE 102010024912
(43) Veröffentlichungstag der Anmeldung: 21.12.2011
(73) Patentinhaber: Aptar Radolfzell GmbH, 78315 Radolfzell (DE)
(72) Erfinder: Kohnle, Jörg, 78056 VS-Schwenningen (DE)
(74) Vertreter: Patentanwälte Ruff, Wilhelm, Beier, Dauster & Partner

(56) Entgegenhaltungen:
- WO-A1-92/17231
- WO-A1-2008/006527
- WO-A2-2005/009325
- US-A- 6 138 669

## Beschreibung

Die Erfindung betrifft eine Inhalationsvorrichtung zur oralen Verabreichung eines pharmazeutischen Mediums. Eine solche gattungsgemäße Inhalationsvorrichtung weist ein Gehäuse mit einem Mundstück, eine in einem Inhalationsbereich des Gehäuses aufgenommene Containereinheit mit einem Mediumcontainer und einem zum Zwecke eines Austragvorgangs gegenüber dem Mediumcontainer verlagerbaren Auslassstutzen mit Auslassöffnung sowie einen Austragsensor zur Erfassung des Mediumaustrags auf.

Gattungsgemäße Inhalationsvorrichtungen werden üblicherweise als "MDI" ("Metered-Dose Inhaler") bzw. "pMDI" ("pressurized Metered-Dose Inhaler") bezeichnet. Sie dienen der Verabreichung von Medikamenten, die zum Zwecke der Behandlung von Atemwegserkrankungen in zerstäubter Form in die Lunge des Benutzers gelangen sollen. Sie weisen das genannte Gehäuse auf, in welches die genannte Containereinheit eingesetzt ist. Das Gehäuse verfügt über einen Lufteinlass, der es gestattet, durch den Inhalationsbereich des Gehäuses hindurch Luft zum Mundstück hin einzusaugen, wobei im Zuge dieses Einsaugens der Austragvorgang von Medium aus dem Mediumcontainer durch Verlagern des Mediumcontainers gegenüber dem Auslassstutzen bewirkt wird, so dass die eingesogene Luft mit dem zerstäubten Medium vermengt und eingeatmet wird und somit in die Lunge gelangen kann.

Der Austrag von Medium aus dem Mediumcontainer wird durch die Relativverlagerung des Mediumcontainers gegenüber dem Auslassstutzen bewirkt. Im einfachsten Falle kann bei gattungsgemäßen Inhalationsvorrichtungen der Mediumcontainer hierzu ein Stück weit aus dem Lufteinlass des Gehäuses herausragen, so dass er unmittelbar manuell kraftbeaufschlagt werden kann, um gegenüber dem in eine korrespondierende Ausnehmung des Gehäuses eingesetzten Auslassstutzen verlagert zu werden.

Gattungsgemäße Inhalationsvorrichtungen sind mit einem Austragsensor zur Erfassung des Austragvorgangs versehen, insbesondere zum Zwecke der Zählung der Austragvorgänge. Hierdurch ist es einem Benutzer möglich, die Zahl der noch im Mediumcontainer verbliebenen Dosen des Mediums zu erfassen und somit einschätzen zu können, wie lange das Medium im Mediumcontainer noch reicht.

Aus dem Stand der Technik sind verschiedene Möglichkeiten zur Gestaltung des Austragsensors bekannt.

Die WO 1997/033640 A1 beschreibt einen Drucksensor, der an einem für den Medienaustrag vorgesehenen Kanal angeordnet ist und den Mediumaustrag dadurch erfassbar macht. Diese Gestaltung ist vergleichsweise komplex und teuer.

Aus der WO 1991/006334 A1 ist eine Gestaltung bekannt, bei der eine mechanische Erfassung des Austragvorgangs dadurch erreicht wird, dass die Bewegung des Mediumcontainers im Inhalationsbereich des Gehäuses erfasst wird. Eine hiermit vergleichbare Lösung ist auch aus der WO 1996/000595 A1 bekannt. Auch die WO 2005/009325 A2 enthält eine Vielzahl von Gestaltungen, die größtenteils ebenfalls vorsehen, dass im Inhalationsbereich des Gehäuses ein Taster angeordnet ist, der im Zuge der Verlagerung des Mediumcontainers im Inhalationsbereich aktiviert wird. Aus diesem Dokument ist es weiterhin auch bekannt, einen Taster an der Außenseite des Gehäuses vorzusehen, der im Zuge der manuellen Kraftbeaufschlagung des Mediumcontainers gegenüber dem Gehäuse unweigerlich manuell mitbetätigt wird.

Weiter im Zusammenhang mit der vorliegenden Erfindung relevante Dokument des Standes der Technik sind die WO 2008/006527 A1 und die WO 92/17231 A1.

Aus der US 6,138,669 A ist eine Gestaltung eines MDI bekannt, bei der der Druck des austretenden Mediums genutzt wird, um einen Flächenabschnitt auszulenken, der mittelbar einen Sensor betätigt.

Die Gestaltungen mit einem Taster, der unmittelbar die Bewegung des Mediumcontainers im Inhalationsbereich erfasst, geht mit dem Nachteil einher, dass eine zuverlässige Erkennung des Mediumaustrags häufig nicht vollständig gewährleistet ist, da die Containereinheit hinsichtlich ihrer Position im Innenbereich des Gehäuses Toleranzen unterliegt, die ein Vorbeiführen des Mediumcontainers am Taster gestatten. Eine dadurch ausbleibende Erkennung eines Austragvorgangs wird grundsätzlich als kritischer als eine fälschlicherweise stattfindende Erkennung eines tatsächlich nicht stattfindenden Austragvorgangs angesehen. Weiterhin ist die Gefahr gegeben, dass der Taster aufgrund seiner unmittelbaren Anordnung im Inhalationsbereich aufgrund von Feuchtigkeit versagt und somit Austragvorgänge nicht zuverlässig erfasst werden.

### Aufgabe und Lösung

Aufgabe der Erfindung ist es, eine gattungsgemäße Inhalationsvorrichtung dahingehend weiterzubilden, dass diese zuverlässig die Austragvorgänge erfassen kann. Dabei soll insbesondere auch eine kostengünstige Gestaltung erreicht werden.

Erfindungsgemäß wird dies durch eine Inhalationsvorrichtung nach Anspruch 1 erreicht.

Erfindungsgemäß ist demnach vorgesehen, dass ein den Inhalationsbereich begrenzender und insbesondere gegenüber einer Umgebung oder einem Aufnahmeraum für elektronische Komponenten trennender Wandungsabschnitt, der Sensorwandungsabschnitt, der eine Aufnahmeausnehmung für den Auslassstutzen aufweist, gegenüber einem Hauptabschnitt des Gehäuses beweglich ist und bei der Verlagerung des Mediumcontainers innerhalb des Inhalationsbereichs bewegt wird. Diese Bewegung des Sensorwandungsabschnitts wird statt der unmittelbaren Erfassung der Bewegung des Mediumcontainers vom Austragsensor erfasst. Dabei ist der Sensorwandungsabschnitt mit dem Hauptabschnitt des Gehäuses einstückig verbunden.

Eine erfindungsgemäße Inhalationsvorrichtung weist die Merkmale der oben beschriebenen gattungsgemäßen Inhalationsvorrichtungen auf. Sie verfügt vorzugsweise über eine Elektronik, die zur Auswertung der Signale des insbesondere als Taster ausgebildeten Austragsensors ausgebildet ist. Diese Elektronik umfasst vorzugsweise einen integrierten Schaltkreis, der insbesondere der Zählung der Austragsvorgänge dient. Weiterhin umfasst sie vorzugsweise eine Batterie als Energiespeicher sowie eine Ausgabeeinheit, insbesondere in Form eines Displays, dem die Zahl der bereits abgegebenen oder noch enthaltenen Dosen zu entnehmen ist.

Der Hauptabschnitt des Gehäuses, demgegenüber der Sensorwandungsabschnitt beweglich ist, umfasst jene Teile der Außenfläche des Gehäuses, die bestimmungsgemäß dem Halten der Inhalationsvorrichtung dienen wie insbesondere einem zylindrischen Gehäuseabschnitt, der die Containereinheit umgibt, sowie alle hierzu ortsfesten Komponenten des Gehäuses, wie insbesondere auch das Mundstück. Der Sensorwandungsabschnitt ist gegenüber diesem Hauptabschnitt beweglich, wobei er derart an den Hauptabschnitt angebunden ist, dass er nach Beendigung eines Austragvorgangs und Wegfall der entsprechenden manuellen Kraftbeaufschlagung in eine Ausgangslage zurückkehrt. Der als Teil des Gehäuses ausgebildete Sensorwandungsabschnitt ist aus dem gleichen Material wie der Hauptabschnitt gefertigt, insbesondere aus Kunststoff, und er ist einstückig zumindest mit den ihn umgebenden Teilen des Hauptabschnitts des Gehäuses verbunden, wobei ein Verbindungsbereich zwischen dem Hauptabschnitt und dem Sensorwandungsabschnitt verformbar ausgebildet ist. Diese Einstückigkeit gestattet insbesondere eine einfache Herstellbarkeit, da im Zuge der Herstellung des Gehäuses der Sensorwandungsabschnitt unmittelbar vorgesehen werden kann. Des Weiteren ist durch diese Einstückigkeit auch die genannte selbsttätige Rückkehr des Sensorwandungsabschnitts in seine Ausgangsposition durch eine entsprechende elastische Gestaltung des Verbindungsbereichs einfach erreichbar. Weiterhin ist die Einstückigkeit auch in Hinblick auf eine gewünschte Flüssigkeitsdichtigkeit von Vorteil. Hierzu ist vorzugsweise weiterhin vorgesehen, dass der Verbindungsbereich den Sensorwandungsabschnitt umlaufend umgibt und dadurch den Sensorwandungsabschnitt flüssigkeitsdichtend mit dem Hauptabschnitt des Gehäuses verbindet. In einem solchen Fall ist der Verbindungsbereich vorzugsweise als Bereich mit gegenüber dem Sensorwandungsabschnitt und dem Hauptabschnitt deutlich verringerter Wandungsstärke ausgebildet. Er kann insbesondere eine leporelloartige Gestaltung aufweisen, um eine unproblematische Verformbarkeit zu gewährleisten. Neben der beschriebenen flüssigkeitsdichtenden Gestaltung mit umlaufenden Verbindungsbereich ist es jedoch auch möglich, die Anbindung des Sensorwandungsabschnitts an den Hauptabschnitt des Gehäuses über diskrete und insbesondere dünnwandige Materialbrücken zu gewährleisten.

Der Austragsensor ist vorzugsweise auf der dem Inhalationsbereich abgewandten Seite des Sensorwandungsabschnitts vorgesehen. Er ist dadurch in einer geschützten Position angeordnet. So ist keine Gefahr gegeben, den Austragsensor beim Einsetzen der Containereinheit zu beschädigen. Insbesondere ist bei der oben beschriebenen flüssigkeitsdichtenden Gestaltung des Verbindungsbereichs auch gewährleistet, dass der dem Inhalationsbereich abgewandte Austragsensor nicht durch im Inhalationsbereich vorhandene Feuchtigkeit beeinträchtigt wird.

Es ist vorgesehen, dass der Sensorwandungsabschnitt im Betrieb stets ortsfest zum Auslassstutzen der Containereinheit verbleibt. Zu diesem Zweck ist es erfindungsgemäß vorgesehen, dass der Sensorwandungsabschnitt eine Aufnahmeausnehmung für den Auslassstutzen aufweist und vorzugsweise eine über einen Kanal mit der Aufnahmeausnehmung verbundene Austragdüse aufweist. Diese Austragdüse mündet dabei in den Inhalationsbereich des Gehäuses und definiert den Bereich, in dem im Betrieb das Medium aus dem Mediumcontainer mit der vom Benutzer eingesogenen Luft vermengt wird. Bei einer solchen Gestaltung, bei der der Sensorwandungsabschnitt ortsfest zum Auslassstutzen verbleibt, ist der Sensorwandungsabschnitt bei üblicher Benutzungsausrichtung eines MDI unterhalb der Containereinheit angeordnet. Er wird dementsprechend beim Hinunterdrücken des Mediumcontainers gegenüber dem Hauptabschnitt des Gehäuses in gleicher Richtung, jedoch in verminderndem Maße, gegenüber dem Hauptabschnitt des Gehäuses verlagert. Eine solche Gestaltung hat sich als besonders zuverlässig erwiesen, da hierdurch ausgeschlossen ist, dass Toleranzen hinsichtlich der Position der Containereinheit im Gehäuse eine inkorrekte Zählung bewirken, sei es Nichtzählung einer tatsächlich stattfindenden Betätigung oder durch Zählung einer begonnenen, jedoch vorzeitig vor Beginn des Flüssigkeitsaustrag beendeten Betätigung.

Der Austragsensor, der Sensorwandungsabschnitt und die Containereinheit sind vorzugsweise derart aufeinander abgestimmt und derart angeordnet, dass im Zuge einer Verlagerung der Containereinheit gegenüber dem Hauptabschnitt des Gehäuses der Sensorwandungsabschnitt den Austragsensor betätigt, sobald oder bevor der Austragvorgang aus dem Mediumcontainer beginnt.

Dies bedeutet, dass die zur Auslenkung des Sensorwandungsabschnitts erforderliche Kraft, die ausreicht, um den Austragsensor mittels des Sensorwandungsabschnitts auszulösen, mit jener Kraft übereinstimmt oder kleiner als jene Kraft ist, die erforderlich ist, um den Auslassstutzen der Containereinheit gegenüber dem Mediumcontainer soweit zu verlagern, dass der Austragvorgang beginnt. Bei der oben genannten Gestaltung, bei der der elastisch gegenüber dem Gehäuse auslenkbare Sensorwandungsabschnitt im Betrieb stets ortsfest zum Auslassstutzen verbleibt, teilt sich eine Verlagerungsstrecke des Mediumcontainers gegenüber dem Hauptabschnitt des Gehäuses auf in eine Teilstrecke, um die der Mediumscontainer gegenüber dem Auslassstutzen und dem Sensorwandungsabschnitt verlagert wird und eine Teilstrecke, um die der Auslassstutzen und der Sensorwandungsabschnitt gegenüber dem Hauptabschnitt des Gehäuses verlagert werden. Durch eine entsprechende Abstimmung der Anbindung des Sensorwandungsabschnitts an den Hauptabschnitt auf die Kraft, die zur Verlagerung des Auslassstutzens gegenüber dem Mediumscontainer erforderlich ist, kann das genannte Verhalten erreicht werden.

Diese Art der Abstimmung gewährleistet, dass auch ein unvollständiger Austragvorgang, beispielsweise aufgrund nicht ausreichender Verlagerung des Mediumcontainers gegenüber dem Hauptabschnitt des Gehäuses, vom Austragsensor registriert wird und somit bei einer Zählung der Austragvorgängen Berücksichtigung findet. Eine Abweichung der aufgrund der Zählung bestimmten Restmenge des Mediums im Mediumcontainer gegenüber der tatsächlich noch vorhandenen Menge des Mediums ist dadurch lediglich in der Form möglich, dass die noch im Mediumcontainer enthaltene Mediummenge unterschätzt wird. Hierdurch wird der Situation vorgebeugt, dass vermeintlich noch Medium in dem Mediumcontainer verbleibt, dieses tatsächlich jedoch nicht mehr vorhanden ist.

Der Austragsensor, der vorzugsweise als Taster ausgebildet ist, kann insbesondere am Hauptabschnitt des Gehäuses vorgesehen sein und so angeordnet sein, dass eine gegenüber einer Basis des Tasters verlagerbare Tasterfläche im Zuge der Betätigung der Inhalationsvorrichtung durch den Sensorwandungsabschnitt niedergedrückt wird. Wie oben bereits erwähnt, ist der Austragsensor vorzugsweise auf der dem Inhalationsbereich abgewandten Seite des Sensorwandungsabschnitts angeordnet. Besonders vorteilhaft ist es, wenn das Gehäuse zwei Teilgehäuse aufweist, die mittels einer Kopplungseinrichtung aneinander ankoppelbar sind, wobei ein erstes Teilgehäuse den Inhalationsbereich umgibt und den Sensorwandungsabschnitt aufweist und wobei ein zweites Teilgehäuse den Austragsensor aufweist.

Bei einer solchen Gestaltung kann das erste Teilgehäuse dafür vorgesehen sein, nach der Verwendung mit einer Containereinheit gemeinsam mit dieser entsorgt zu werden, während das zweite Teilgehäuse, welches den Austragsensor sowie vorzugsweise alle anderen elektronischen Komponenten der Inhalationsvorrichtung aufweist, wiederverwendet wird. In einem solchen Falle ist der Austragsensor am zweiten Teilgehäuse so angeordnet, dass eine Kraftbeaufschlagung von außen den Sensor auslösen kann, wobei dieses Kraftbeaufschlagung im gekoppelten Zustand der Teilgehäuse durch den entsprechend angeordneten Sensorwandungsabschnitt bewirkt wird.

Bei einer Weiterbildung dieser Gestaltung mit zwei Teilgehäusen weist das zweite Teilgehäuse einen Sensor auf, der geeignet ist, den Kopplungszustand der beiden Teilgehäuse zu erfassen. Dies kann ein separater Sensor sein. Es kann jedoch auch der Austragsensor so ausgebildet sein, dass er in Abhängigkeit beispielsweise der Auslenkung seiner Tasterfläche zum einen den Kopplungszustand und zum anderen die Verlagerung des Sensorwandungsabschnitts erfassen kann.

Vorzugsweise sind die elektronischen Komponenten, insbesondere der zur Zählung vorgesehene Schaltkreis, derart ausgebildet, dass eine Betätigung des zur Zählung vorgesehenen Sensors nur dann für die Zählung herangezogen wird, wenn die Teilgehäuse sich im gekoppelten Zustand befinden.

Bei einer solchen Gestaltung mit zwei Teilgehäusen ist es bevorzugst, dass das zweite Teilgehäuse einen Innenbereich aufweist, der im Bereich des Austragsensors durch einen formveränderlichen oder verlagerbaren Wandungsabschnitt begrenzt ist. Dies gestattet es, die Elektronik vollständig zu kapseln. Der Austragsensor liegt in einem solchen Falle auch im entkoppelten Zustand der Teilgehäuse nicht ungeschützt an der Außenseite des zweiten Teilgehäuses, sondern ist durch diesen Wandungsabschnitt, beispielsweise durch eine dünnwandige Membran, geschützt.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches nachfolgend anhand der Figuren erläutert wird. Dabei zeigen:
- Fig. 1: eine erfindungsgemäße Inhalationsvorrichtung vor der Betätigung und
- Fig. 2: die erfindungsgemäße Inhalationsvorrichtung während der Betätigung.

### Detaillierte Beschreibung des Ausführungsbeispiels

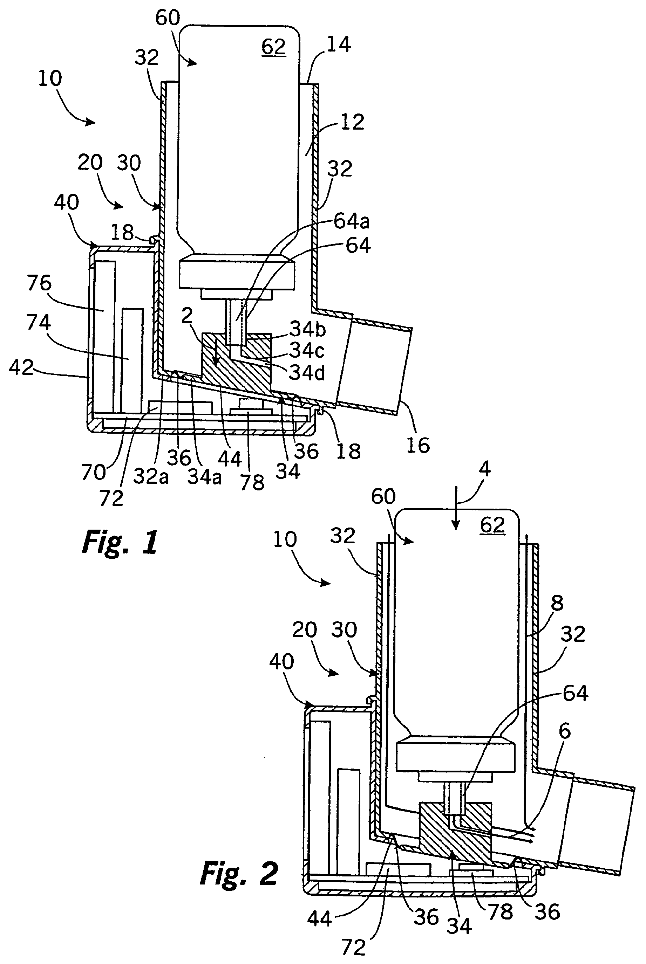

Fig. 1 zeigt eine erfindungsgemäße Inhalationsvorrichtung 10 vor deren Betätigung.

Die Inhalationsvorrichtung 10 verfügt über ein Gehäuse 20, welches sich aus zwei Teilgehäusen 30, 40 zusammensetzt, die mittels Kopplungseinrichtung 18 werkzeuglos verbindbar und trennbar sind.

Das erste Teilgehäuse 30 weist in etwa die Form eines konventionellen MDI auf. Es umgibt einen etwa L-förmigen Inhalationsbereich 12, dessen oberes Ende offen gestaltet ist und ein Lufteinlass 14 bildet und dessen unteres Ende ebenfalls offen gestaltet ist und ein Mundstück 16 bildet. Die Außenwandungen 32, 34 dieses ersten Teilgehäuses 30 sind mit Ausnahme eines Sensorwandungsabschnitts 34 zueinander und zu den Wandungen des zweiten Teilgehäuses 40 im Betrieb ortsfest und bilden den Hauptabschnitt 32 des Teilgehäuses 30. Lediglich der genannte Sensorwandungsabschnitt 34, der am unteren Ende des ersten Teilgehäuses 30 vorgesehen ist, ist gegenüber diesem Hauptabschnitt 32 des ersten Teilgehäuses 30 beweglich, wobei dies dadurch erreicht wird, dass der Sensorwandungsabschnitt 34 mittels eines umlaufenden dünnwandigen und leporelloartig gewellten Verbindungsbereichs 36 mit dem Hauptabschnitt 32 einstückig verbunden ist. Dies gestattet eine Relativverlagerung des Sensorwandungsabschnitts 34 in Richtung des Pfeils 2.

Der Sensorwandungsabschnitt 34 weist einen flachen Randbereich 34a auf, der mit den umgebenden Bodenabschnitten 32a des Hauptabschnitts 32, mit denen er einstückig verbunden ist, fluchtet. In der Mitte des Sensorwandungsabschnitts 34 ist dieser verdickt ausgebildet, wobei in dieser Verdickung eine zylindrische Aufnahmeausnehmung 34b und ein sich davon erstreckender Kanal 34c vorgesehen sind, der in einer Düsenöffnung 34d mündet.

In den Inhalationsbereich 12 des ersten Teilgehäuses 30 ist eine Containereinheit 60 eingesetzt. Diese verfügt über einen Mediumcontainer 62 sowie einen Auslassstutzen 64 mit einer Auslassöffnung 64a. Zum Zwecke des Mediumsaustrags aus dem Mediumcontainer 62 kann der Auslassstutzen gegen eine Gegenkraft gegenüber dem Mediumcontainer 62 verlagert werden. Im in Fig. 1 dargestellten Zustand, in dem die Containereinheit bereits in das erste Teilgehäuse 30 eingesetzt ist, ist der Auslassstutzen 64 in die Aufnahmeausnehmung 34b des Sensorwandungsabschnitts 34 eingesetzt. Hierbei können der Auslassstutzen 64 und die Aufnahmeausnehmung 34b derart aufeinander angepasst sein, dass sie eine kraftschlüssige Klemmverbindung eingehen. Das obere Ende des Mediumcontainers 62 ragt durch den Lufteinlass 14 hindurch nach oben aus dem ersten Teilgehäuse 30 heraus, wobei der herausragende Teil zur Betätigung der Inhalationsvorrichtung 10 vorgesehen ist.

Das zweite Teilgehäuse 40 umfasst alle elektronischen Komponenten der Inhalationsvorrichtung 10, wobei diese in den Figuren nur schematisch dargestellt sind. Die elektronischen Komponenten umfassen eine Basisplatine 70, auf der ein integrierter Schaltkreis 72, ein Energiespeicher in Form einer Batterie 74 sowie eine LCD-Anzeige 76 vorgesehen sind. Die LCD-Anzeige 76 ist dabei so positioniert, dass sie durch eine Ausnehmung 42 in der Wandung des zweiten Teilgehäuses 40 hindurch abgelesen werden kann. Im Bereich des Sensorwandungsabschnitts 34 weist das zweite Teilgehäuse 40 eine Durchbrechung 44 auf. Unterhalb dieser Durchbrechung 44 ist auf der Basisplatine 70 ein Taster 78 angeordnet.

Die genannten elektronischen Komponenten 70, 72, 74, 76 sind dafür ausgebildet, Betätigungen des Tasters 78 zu erfassen und zu zählen, wobei das Ergebnis dieser Zählung auf dem Display 76 dargestellt wird. Sie dienen somit gemeinsam der Zählung der erfolgten Austragvorgänge.

Die Funktionsweise ist dabei die Folgende: Zur Verwendung der Inhalationsvorrichtung wird bestimmungsgemäß das Mundstück 16 von den Lippen des Benutzers umschlossen und Luft angesogen. Gleichzeitig wird der Mediumcontainer 62 manuell gegenüber dem Hauptabschnitt 32 des ersten Teilgehäuses 30 in Richtung des Pfeils 4 hinabgedrückt. Wie in Fig. 2 dargestellt ist, führt dies einerseits dazu, dass der Auslassstutzen 64 gegenüber dem Mediumcontainer 62 eingedrückt wird. Darüber hinaus wird bei gleichzeitiger elastischer Auslenkung des Verbindungsbereichs 36 auch der Sensorwandungsabschnitt 34 des ersten Teilgehäuses 30 hinabgedrückt, wobei er durch die Ausnehmung 44 des zweiten Teilgehäuses 40 in dieses eindringt und dabei in Kontakt mit dem Taster 78 gelangt. Die fortgesetzte Verlagerung des Mediumcontainers 62 bewirkt somit, dass in etwa gleichzeitig ein Betätigungssignal vom Taster 78 an den integrierten Schaltkreis 72 gesendet wird und der Mediumaustrag beginnt, im Zuge dessen Medium entlang des Pfeils 6 aus dem Mediumcontainer 62 in den Inhalationsbereich 12 gelangt, wo es sich mit der vom Benutzer eingesogenen Luft, die entlang der Pfeile 8 strömt, vermengt und durch das Mundstück 16 vom Benutzer eingesogen wird.

Wie bereits beschrieben, wird der Austragvorgang aufgrund der Betätigung des Betätigungstasters 78 vom integrierten Schaltkreis 72 erfasst und auf der LCD-Anzeige 76 wiedergegeben. Dabei kann sowohl vorgesehen sein, dass auf der LCD-Anzeige 76 die noch im Container verbleibenden Dosen dargestellt werden oder aber die bereits entnommenen Dosen dargestellt werden.

Sobald alle Dosen im Mediumcontainer 62 verbraucht sind, können die Teilgehäuse 30, 40 voneinander entkoppelt werden und ein neues erstes Teilgehäuse 30 mit einer neuen Containereinheit 60 an das wieder verwendbare zweite Teilgehäuse 40 mit der Elektronik 70, 72, 74, 76, 78 angekoppelt werden.

Bei einer nicht dargestellten Weiterbildung kann die Öffnung 44 des zweiten Teilgehäuses 40 auch durch eine Membran oder einen in ähnlicher Weise wie der Sensorwandungsabschnitt 34a ausgebildeten Wandungsabschnitt verschlossen sein, so dass auch das isolierte zweite Teilgehäuse 40 nach außen hermetisch abgeschlossen ist.

## Patentansprüche

1. Inhalationsvorrichtung (10) zur oralen Verabreichung eines pharmazeutischen Mediums mit
- einem Gehäuse (20) mit einem Mundstück (16),
- einer in einem Inhalationsbereich (12) des Gehäuses (20) aufgenommenen Containereinheit (60) mit
- einem Mediumcontainer (62) und
- einem zum Zwecke eines Austragvorgangs gegenüber dem Mediumcontainer (62) verlagerbaren Auslassstutzen (64) mit Auslassöffnung (64a),
und
- einem Austragssensor (78) zur Erfassung des Austragvorgangs,
**dadurch gekennzeichnet, dass**
- das Gehäuse (20) einen Hauptabschnitt (32) und einen gegenüber dem Hauptabschnitt (32) verlagerbaren Sensorwandungsabschnitt (34) aufweist,
- der mit dem Hauptabschnitt (32) des Gehäuses (20) einstückig verbunden ist, wobei ein Verbindungsbereich (36) zwischen dem Hauptabschnitt (32) und dem Sensorwandungsabschnitt (34) verformbar ausgebildet ist, und
- der im Betrieb stets ortsfest zum Auslassstutzen (64) verbleibt und hierfür eine Aufnahmeausnehmung (34b) für den Auslassstutzen (64) aufweist,
so dass der Sensorwandungsabschnitt (34) während eines Austragvorgangs gegenüber dem Hauptabschnitt (32) verlagert wird,
- der Austragssensor (78) zur Erfassung der Verlagerung des Sensorwandungsabschnitts (34) gegenüber dem Hauptabschnitt (32) des Gehäuses (20) ausgebildet ist.

2. Inhalationsvorrichtung nach Anspruch 1,
**dadurch gekennzeichnet, dass**
der Verbindungsbereich (36) den Sensorwandungsabschnitt (34) umlaufend umgibt und dadurch den Sensorwandungsabschnitt (34) flüssigkeitsdichtend mit dem Hauptabschnitt (32) des Gehäuses (20) verbindet.

3. Inhalationsvorrichtung nach einem der vorstehenden Ansprüche,
**dadurch gekennzeichnet, dass**
der Austragssensor (78) auf der dem Inhalationsbereich (12) abgewandten Seite des Sensorwandungsabschnitts (34) vorgesehen ist.

4. Inhalationsvorrichtung nach einem der vorstehenden Ansprüche,
**dadurch gekennzeichnet, dass**
der Sensorwandungsabschnitt (34) einerseits und der Auslassstutzen (64) andererseits zueinander ortsfest vorgesehen sind.

5. Inhalationsvorrichtung nach einem der vorstehenden Ansprüche,
**dadurch gekennzeichnet, dass**
der Sensorwandungsabschnitt (34) eine über einen Kanal (34c) mit der Aufnahmeausnehmung (34b) verbundene Austragdüse (34d) aufweist.

6. Inhalationsvorrichtung nach einem der vorstehenden Ansprüche,
**dadurch gekennzeichnet, dass**
der Austragssensor (78), der Sensorwandungsabschnitt (34) und die Containereinheit (60) derart aufeinander abgestimmt und angeordnet sind, dass im Zuge einer Verlagerung der Containereinheit (60) gegenüber dem Hauptabschnitt (32) des Gehäuses (20) der Sensorwandungsabschnitt (34) den Austragssensor (78) betätigt, sobald oder bevor der Austragvorgang beginnt.

7. Inhalationsvorrichtung nach einem der vorstehenden Ansprüche, **dadurch gekennzeichnet, dass**
das Gehäuse (20) zwei Teilgehäuse (30, 40) aufweist, die mittels einer Kopplungseinrichtung (18) aneinander ankoppelbar sind, wobei
- ein erstes Teilgehäuse (30) den Inhalationsbereich (12) umgibt und den Sensorwandungsabschnitt (34) aufweist und
- ein zweites Teilgehäuse (40) den Austragssensor (78) aufweist.

8. Inhalationsvorrichtung nach Anspruch 7,
**dadurch gekennzeichnet, dass**
das zweite Teilgehäuse einen Innenbereich aufweist, der im Bereich des Austragssensors durch einen formveränderlichen oder verlagerbaren Wandungsabschnitt begrenzt ist.

## Claims

1. An inhaler device (10) for oral administration of a pharmaceutical medium, comprising
- a housing (20) having a mouthpiece (16),
- a container unit (60) accommodated in an inhalation region (12) of said housing (20) and including
- a medium container (62) and
- an outlet connector (64) having an outlet orifice (64a), said outlet connector is capable of being moved relatively to said medium container (62) for the purpose of a discharging operation,
and
- a discharge sensor (78) for the detection of a discharging operation, **characterized in that**
- said housing (20) has a main portion (32) and a sensor wall portion (34) that is capable of being moved relatively to said main portion (32),
- said sensor wall portion (34) is integrally connected to said main portion (32) of said housing (20) and a connecting region (36) between said main portion (32) and said sensor wall portion (34) is deformable, and
- said sensor wall portion (34) during operation remains fixed in location relative to the outlet connector (64) and for this purpose has an accommodation recess (34b) for said outlet connector (64),
with the result that the sensor wall portion (34) is displaced relative to said main portion (32) during a discharging operation,
- said discharge sensor (78) is adapted to detect the displacement of the sensor wall portion (34) in relation to said main portion (32) of said housing (20).

2. The inhaler device according to claim 1, **characterized in that** said connecting region (36) surrounds said sensor wall portion (34) around its periphery and by this means connects said sensor wall portion (34) to said main portion (32) of said housing (20) in a liquid-tight manner.

3. The inhaler device according to any one of the preceding claims, **characterized in that** said discharge sensor (78) is provided on that side of said sensor wall portion (34) that is facing away from said inhalation region (12).

4. The inhaler device according to any one of the preceding claims, **characterized in that** the sensor wall portion (34) on the one hand and the outlet connector (64) on the other hand are fixed in location with respect to each other.

5. The inhaler device according to any one of the preceding claims, **characterized in that** the sensor wall portion (34) has a discharge nozzle (34d) connected to the accommodation recess (34b) via a channel (34c).

6. The inhaler device according to any one of the preceding claims, **characterized in that** said discharge sensor (78), said sensor wall portion (34), and said container unit (60) are adapted to each other and disposed such that when there is a displacement of said container unit (60) relative to said main portion (32) of said housing (20), said sensor wall portion (34) actuates said discharge sensor (78) as soon as, or before, the discharging operation commences.

7. The inhaler device according to any one of the preceding claims, **characterized in that** the housing (20) has two sub-housings (30, 40) which are capable of being coupled together by means of a coupling device (18), and wherein
- a first sub-housing (30) surrounds said inhalation region (12) and comprises said sensor wall portion (34) and
- a second sub-housing (40) comprises said discharge sensor (78).

8. The inhaler device according to claim 7, **characterized in that** the second sub-housing has an interior region which is delimited in the region of said discharge sensor by a deformable or movable wall portion.

## Revendications

1. Dispositif d'inhalation (10) pour l'administration orale d'un produit pharmaceutique, comprenant
- un boîtier (20) muni d'une embouchure (16),
- une unité de récipient (60) logée dans une région d'inhalation (12) du boîtier (20), comprenant
- un récipient de produits (62) et
- une tubulure de sortie (64) avec une ouverture de sortie (64a) pouvant être déplacée par rapport au récipient de produits (62) pour effectuer une opération de déchargement,
et
- un capteur de déchargement (78) pour détecter l'opération de déchargement,
**caractérisé en ce que**
- le boîtier (20) présente une portion principale (32) et une portion de paroi de capteur (34) déplaçable par rapport à la portion principale (32),
- laquelle est connectée d'une seule pièce à la portion principale (32) du boîtier (20), une région de connexion (36) étant réalisée de manière déformable entre la portion principale (32) et la portion de paroi de capteur (34), et
- laquelle, pendant le fonctionnement, reste toujours fixe par rapport à la tubulure de sortie (64), et présente à cet effet un évidement de réception (34b) pour la tubulure de sortie (64),
de telle sorte que la portion de paroi de capteur (34), pendant une opération de déchargement, soit déplacée par rapport à la portion principale (32),
- le capteur de déchargement (78) est réalisé de manière à détecter le déplacement de la portion de paroi de capteur (34) par rapport à la portion principale (32) du boîtier (20).

2. Dispositif d'inhalation selon la revendication 1,
**caractérisé en ce que**
la région de connexion (36) entoure sur sa périphérie la portion de paroi de capteur (34) et relie de ce fait la portion de paroi de capteur (34) de manière étanche aux liquides à la portion principale (32) du boîtier (20).

3. Dispositif d'inhalation selon l'une quelconque des revendications précédentes,
**caractérisé en ce que**
le capteur de déchargement (78) est prévu sur le côté de la portion de paroi de capteur (34) opposé à la région d'inhalation (12).

4. Dispositif d'inhalation selon l'une quelconque des revendications précédentes,
**caractérisé en ce que**
la portion de paroi de capteur (34) d'une part et la tubulure de sortie (64) d'autre part sont prévues de manière fixe l'une par rapport à l'autre.

5. Dispositif d'inhalation selon l'une quelconque des revendications précédentes,
**caractérisé en ce que**
la portion de paroi de capteur (34) présente une buse de déchargement (34d) connectée à l'évidement de réception (34b) par le biais d'un canal (34c).

6. Dispositif d'inhalation selon l'une quelconque des revendications précédentes,
**caractérisé en ce que**
le capteur de déchargement (78), la portion de paroi de capteur (34) et l'unité de récipient (60) sont adaptés les uns aux autres et sont disposés de telle sorte qu'au cours d'un déplacement de l'unité de récipient (60) par rapport à la portion principale (32) du boîtier (20), la portion de paroi de capteur (34) actionne le capteur de déchargement (78), dès ou avant le début de l'opération de déchargement.

7. Dispositif d'inhalation selon l'une quelconque des revendications précédentes,
**caractérisé en ce que**
le boîtier (20) présente deux boîtiers partiels (30, 40), lesquels peuvent être accouplés l'un à l'autre au moyen d'un dispositif d'accouplement (18),
- un premier boîtier partiel (30) entourant la région d'inhalation (12) et présentant la portion de paroi de capteur (34) et
- un deuxième boîtier partiel (40) présentant le capteur de déchargement (78).

8. Dispositif d'inhalation selon la revendication 7,
**caractérisé en ce que**
le deuxième boîtier partiel présente une région intérieure qui est limitée dans la région du capteur de déchargement par une portion de paroi pouvant être déplacée ou de forme variable.
